# DEMANDE DE BREVET EUROPEEN

(11) **EP 0 910 987 A1**
(43) Date de publication de la demande: **28.04.1999**
(21) Numéro de dépôt: 98402574.2
(22) Date de dépôt: 16.10.1998
(51) Int. Cl.: A61B 5/117

(54) **Dispositif et procede de saisie d'empreintes digitales**

(30) Priorité: 17.10.1997 FR 9713031
(71) Demandeur: THOMSON-CSF, 75008 Paris (FR)
(72) Inventeur: Charbonnel, Marie-Pierre, Thomson-CSF P.I.D.P.Cons, 94117 Arcueil cedex (FR); Bricot, Claude, Thomson-CSF P.I.Dept.Prot.Conseil, 94117 Arcueil cedex (FR)

(57) **Abrégé**

Dispositif de saisie d'empreintes digitales comprenant:
- un bloc support (1) muni à sa face supérieure (12) d'au moins une alvéole (3) de forme allongée selon un axe (OX);
- un dévideur (2) de ruban autocollant (4) situé à une première extrémité (10) du bloc (1) et permettant le dévidage du ruban (4) autocollant perpendiculairement à l'axe de l'alvéole (3), la face non collante du ruban reposant sur le bloc au-dessus de l'alvéole.

Le procédé consiste à enduire d'un pigment coloré chaque doigt dont on veut saisir l'empreinte. On applique ensuite chaque doigt sur la partie collante du ruban (4) et on forme ainsi l'empreinte sur cette partie collante. Le ruban (4) est ensuite appliqué sur un support papier et l'empreinte se trouve transposée sur le support tout en étant protégée par le ruban.

Application : Prise d'empreintes digitales.

## Description

L'invention concerne un dispositif et un procédé de saisie d'empreintes digitales et notamment un dispositif utilisant un pigment ou une encre qui, appliquée sur les doigts de la main, peut être transférée sur un support.

Un certain nombre de système de saisie mécanique d'empreintes utilise un support de papier imprimé par de l'encre ou par une substance colorante généralement humide s'en rapprochant. Certains de ces systèmes utilisent également des papiers se colorant au contact d'une substance chimique appliquée sur les doigts.

Ces systèmes présentent l'inconvénient de nécessiter la pression du doigt dont on doit saisir l'empreinte sur un support ce qui déforme l'empreinte. Ils nécessitent également une rotation (ou roulée) du doigt sur le support ce qui ne permet pas d'assurer une répétitivité de la prise d'empreinte.

D'autres capteurs sont basés sur des principes d'optique et capturent l'image de l'empreinte à l'aide des prismes réfléchissant des rayons lumineux.

Une quantité importante des capteurs d'empreintes existants capture une image de l'empreinte digitale à l'aide d'une caméra classique ou à infrarouge.

Enfin, une autre catégorie de capteurs est basée sur l'application de propriétés électro-capacitives.

Ces capteurs sont plus coûteux et présentent pour certains des risques de déformation des empreintes.

L'invention permet de résoudre ces inconvénients.

L'invention concerne donc un dispositif de saisie d'empreintes digitales comprenant :
- un bloc support muni à sa face supérieure d'au moins une alvéole de forme allongée selon un axe ;
- un dévideur de ruban adhésif situé à une première extrémité du bloc et permettant le dévidage du ruban autocollant perpendiculairement à l'axe de l'alvéole, la face non collante du ruban reposant sur le bloc au-dessus de l'alvéole.

Les différents objets et caractéristiques de l'invention apparaîtront dans la description qui va suivre et dans les figures annexées qui représentent :
- la figure 1, une vue en perspective d'un exemple de réalisation simplifié du dispositif de l'invention ;
- les figures 2a et 2b, des vues en perspectives d'exemples de réalisation plus détaillé du dispositif de l'invention ;
- la figure 3, une vue explicative d'utilisation du dispositif de la figure 2a ;
- les figures 4 et 5, des exemples de réalisation du dispositif de découpe du ruban ;
- les figures 6a et 6b, une variante de réalisation de l'invention ;
- les figures 7a à 7c, un système de mise en oeuvre de l'invention pour l'application d'empreintes digitales sur un document.

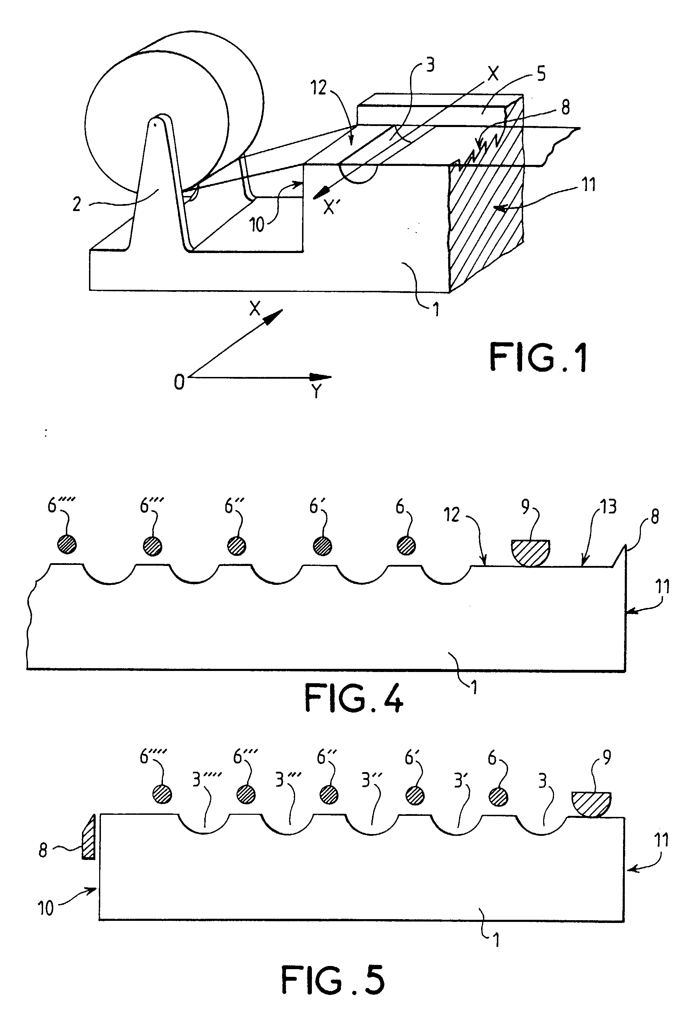

En se reportant à la figure 1, on va tout d'abord décrire un exemple de réalisation simplifié du dispositif de l'invention.

Ce dispositif comporte un bloc support 1 muni à sa face supérieure 12 d'une alvéole 3 (ou goulotte) de forme allongée orientée selon l'axe OX. A une extrémité 10 du bloc est prévu un dévidoir 2 de ruban autocollant 4 de type papier adhésif. Le dévidoir est orienté de façon que le ruban 4 puisse se dérouler selon un axe OY sensiblement perpendiculaire à l'axe OX. De plus, la bobine de ruban 4 est montée dans le dévidoir de façon que la face non collante du ruban soit en contact avec la face 12 du bloc support et recouvre l'alvéole 3.

De cette façon pour prendre l'empreinte digitale d'un doigt on déroule le ruban 4 et on le place sur la face 12 au-dessus de l'alvéole 3. Ensuite, après avoir enduit un doigt d'une encre ou d'un pigment, on applique ce doigt sur le ruban 4 à l'emplacement de l'alvéole 3 de façon que le doigt appuie dans le fond de l'alvéole et que le ruban 4 entoure partiellement le doigt. L'encre ou le pigment du doigt va être transféré sur le ruban, on réalise ainsi la prise d'empreinte. Le doigt étant retiré, il suffit de découper la partie de ruban portant l'empreinte.

De plus, si le ruban est transparent, on peut le coller sur un support, papier par exemple, et l'empreinte prise se trouve enfermée entre ce support papier et le ruban lui-même ce qui met l'empreinte à l'abri de toute détérioration.

Le dispositif de la figure 1 peut être complété par une butée 5 permettant d'appuyer l'extrémité du doigt et donc de fixer la position du doigt sur le ruban 4.

De plus l'extrémité 11 du bloc support 1 opposée à l'extrémité 10 peut comporter un dispositif de coupe 8 du ruban tel qu'une forme dentelée sur laquelle l'utilisateur peut découper le ruban en tirant sur le ruban vers le bas. Le dispositif de coupe peut être avantageusement une lame dentelée qui émerge légèrement de la face supérieure 12 du bloc support.

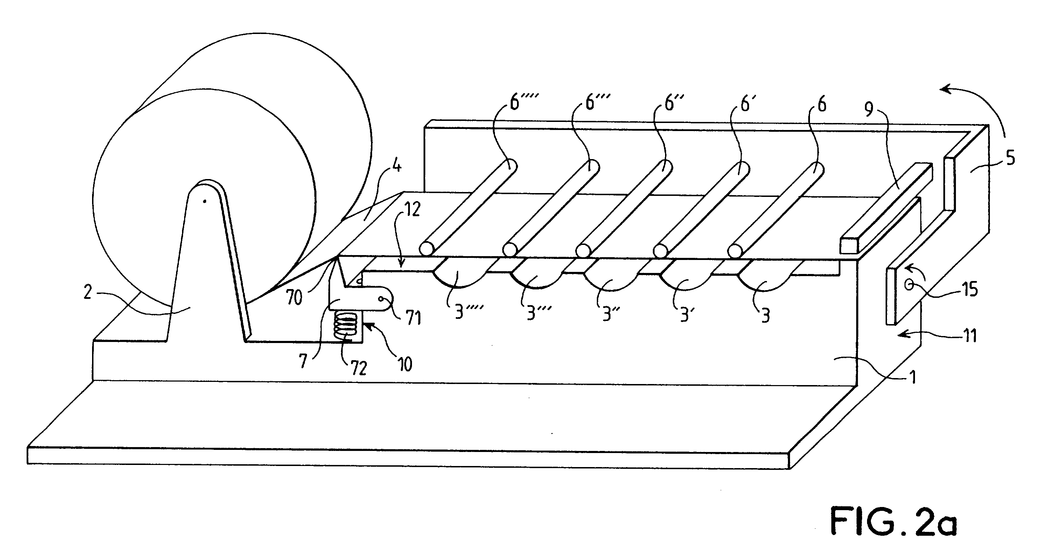

La figure 2a, représente un exemple de réalisation plus détaillé du dispositif de l'invention. Selon cette forme de réalisation le bloc support comporte cinq alvéoles 3, 3', 3", 3"' et 3"" permettant la prise d'empreintes des cinq doigts d'une main. Ces cinq alvéoles sont disposées parallèlement le long du bloc support selon la direction OY de déroulement du ruban 4.

De part et d'autre de chaque alvéole sont prévues des barres de séparation telles que 6, 6', etc.. Ces barres sont situées au-dessus du ruban 4 et permettent au ruban de glisser entre les barres et la face 12. Les barres ont pour rôle de positionner chaque doigt dans une alvéole et permettre le décollement du doigt à partir du ruban lorsqu'on retire le doigt.

Sur la figure 2a la pièce de butée 5 est amovible. Par exemple elle est mobile en rotation autour d'un axe 15. Les barres de séparation sont fixées sur la pièce de butée 5. De cette façon, la rotation de la pièce de butée 5 permet de dégager les barres de séparation pour permettre la mise en place du ruban 4.

A l'extrémité 11 du bloc support est prévu un dispositif 9 de maintien du ruban qui a pour rôle de coincer le ruban entre la pièce 9 et la face 12 du bloc support. Dans le cas où la pièce de butée 5 est amovible, le dispositif de maintien 9 peut être solidaire de la pièce de butée et lorsque la pièce de butée est mise en place pour la prise d'empreintes, le dispositif 9 vient maintenir le ruban.

A l'extrémité 12 est prévu un dispositif de tension élastique 7. Il comporte une pièce de tension 7 articulée autour d'un axe 71 et qui sous l'effet du ressort 72 appuie par son arête 70 sur le ruban 4.

Le dévidoir 2 de ruban comporte un dispositif de freinage ou de blocage de déroulement constitué par exemple d'un écrou de serrage qui agit sur le dévidoir par l'intermédiaire d'une rondelle de freinage.

Lors d'une prise d'empreintes, le ruban est disposé sur la face 12 (face non collante sur la face 12) avec une extrémité du ruban maintenue par le dispositif de maintien 9. Le dévideur 2 est bloqué ou freiné. Le dispositif de tension est en position haute sous l'effet du ressort 72.

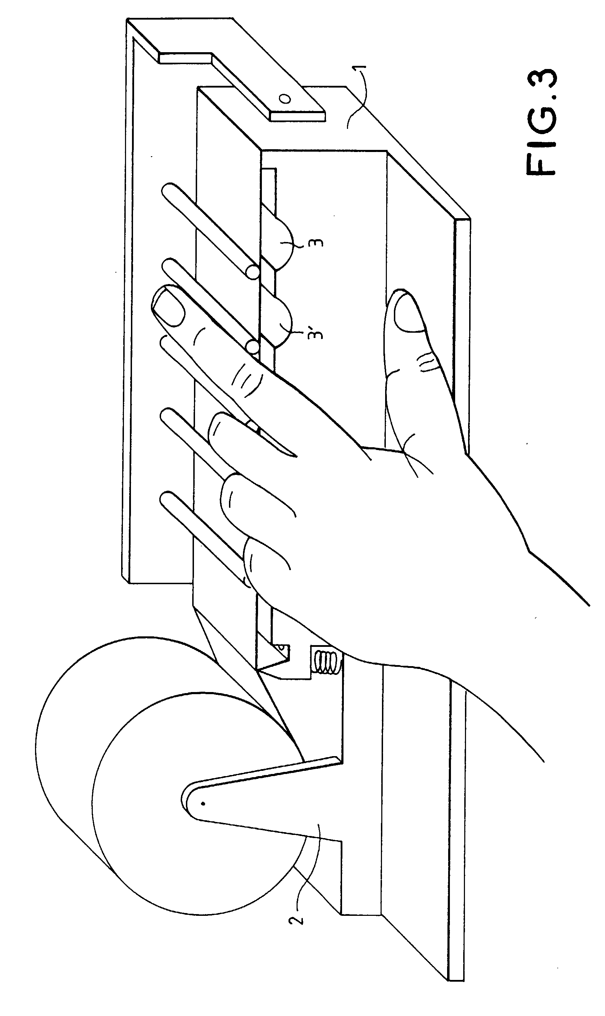

Lorsqu'on place un doigt dans une alvéole 3 à 3"" (voir figure 3), et qu'on appuie le doigt jusqu'au fond de l'alvéole, le ruban se déplace et se tend légèrement ce qui est possible en raison du fait que le tendeur 7 se déplace vers le bas. Lorsqu'on retire le doigt, le ruban se tend à nouveau sous l'effet du ressort 72 et du tendeur 7 et permet la prise d'empreinte d'un autre doigt dans une autre alvéole.

La saisie des empreintes d'une main se déroule donc de la façon suivante : l'utilisateur colore le premier doigt à l'aide du pigment préalablement étalé sur un morceau de tissu ou de buvard. Il applique ensuite ce doigt sur le morceau de ruban 4 correspondant à la première alvéole. Il décolle ensuite son doigt du ruban et recommence l'opération pour les quatre doigts restants.

Le support métallique 5 se soulève manuellement permettant de récupérer la bande de ruban sur laquelle sont imprimées les cinq dessins d'empreintes. Celle-ci peut être alors déposée sur le formulaire où elle adhère tout naturellement.

Après avoir pris toutes les empreintes nécessaires, on soulève donc la pièce de butée 5, les barres de séparation 3 à 3"" et le dispositif de maintien 9. On tire l'extrémité libre du ruban jusqu'à ce que la zone du ruban située à l'extrémité 10 arrive à l'extrémité 11 et on coupe la portion de ruban portant les empreintes.

Cette portion de ruban est ensuite collée sur un support papier, les empreintes digitales se trouvant protégées par le ruban contre toute détérioration extérieure.

La découpe du ruban peut se faire à l'extrémité 11 du bloc support après avoir rabattu la pièce de butée 5, une nouvelle portion de ruban étant en place sur la face 12 du bloc support et étant maintenue par la pièce de maintien 9.

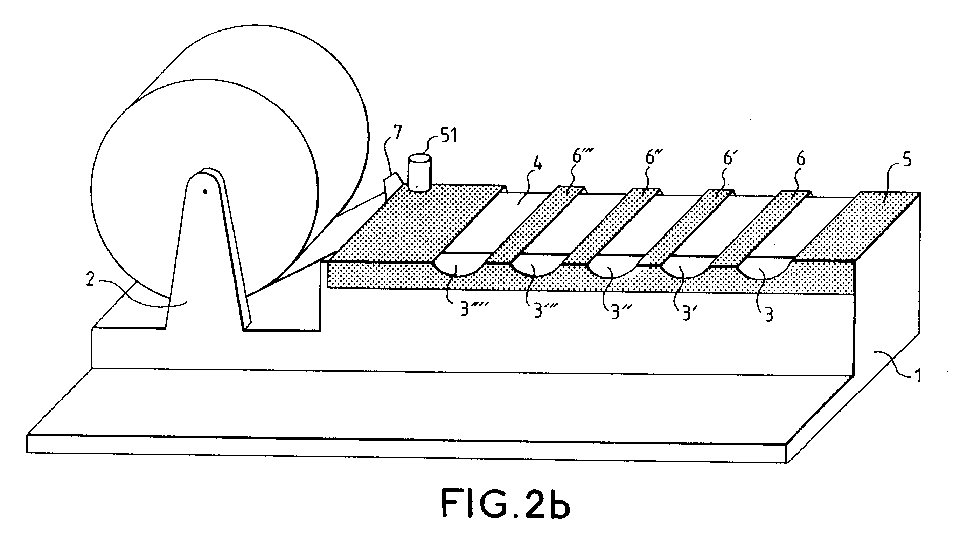

La figure 2b représente une variante de réalisation de la figure 2a. Dans cette variante, la pièce de butée 5 est articulée autour d'un axe situé en bout du bloc support 1 et perpendiculairement à l'axe du ruban 4. Un dispositif de coupe du ruban est situé du côté de l'extrémité libre de la pièce butée 5. Une poignée (ou bouton) 51 permet de soulever la pièce butée 5.

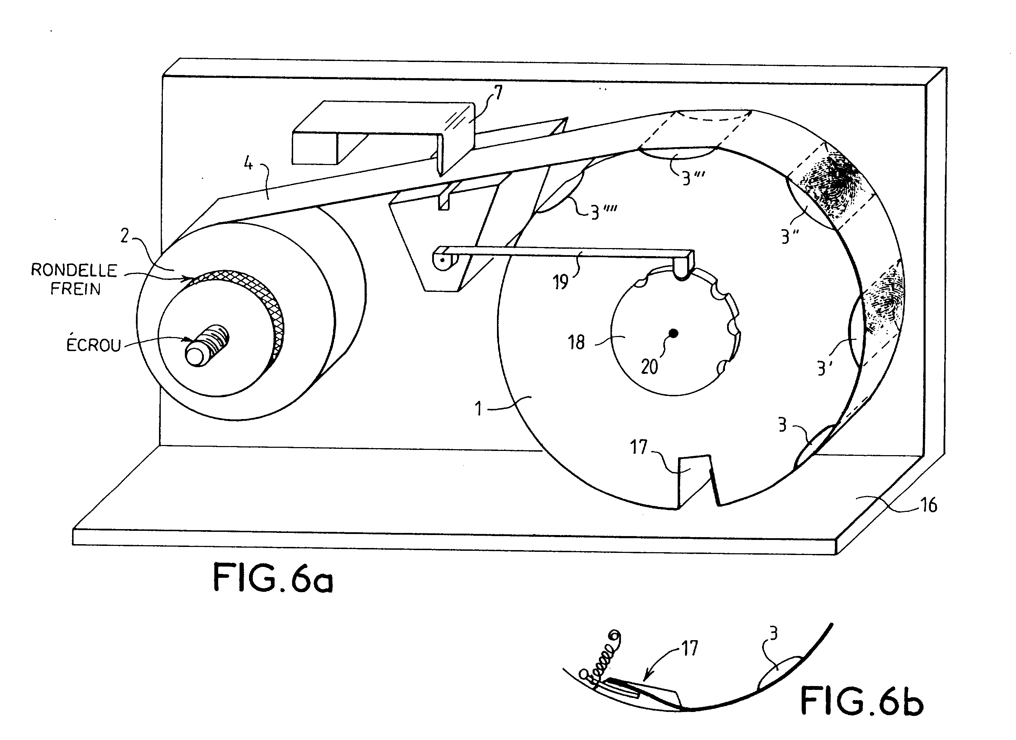

La figure 4 représente un exemple de réalisation du dispositif de découpe. L'extrémité 11 du bloc support est terminée par une arête tranchante. Par exemple, cette arête tranchante peut être réalisée à l'aide d'une denture ou d'une lame dentée. Entre cette arête tranchante et le dispositif de maintien 9 est prévue une zone 13 de longueur environ 1 à 2 cm permettant de maintenir le ruban sur la face 10 lorsqu'on soulève le dispositif de maintien et permettant d'appréhender l'extrémité du ruban pour le trier et pour le découper.

Selon une variante de réalisation, le dispositif de découpe du ruban peut être aménagé du côté de l'extrémité 10 du bloc support. Comme représenté en figure 5, ce dispositif peut comporter une lame tranchante, telle qu'une lame dentelée, solidaire de la butée amovible 5. De cette façon, en soulevant la butée amovible on découpe le ruban 4 et on le rend accessible pour qu'on puisse le prendre et le coller sur une feuille de papier.

De façon plus générale, on voit donc que le procédé de l'invention prévoit d'enduire d'un pigment le doigt dont on désire prendre l'empreinte digitale, puis de l'appliquer sur la face collante d'un ruban adhésif transparent et enfin de coller ce ruban adhésif sur un support papier.

Pour saisir l'empreinte en obtenant l'équivalent d'une empreinte "roulée", le ruban est disposé sur une forme en creux ayant sensiblement la forme du doigt, la face non collante étant en contact avec la forme en creux.

L'avantage principal du système de l'invention par rapport aux systèmes existants est la garantie d'une qualité de saisie. En effet, le ruban autocollant en adhérant au doigt permet d'éviter la distorsion des lignes composant l'empreinte que l'on obtient habituellement avec un support rigide. De plus, en utilisant de préférence un pigment on évite les bavures que l'on observe avec des colorants liquides de type encre.

En outre, grâce aux alvéoles cylindriques creusées dans le support de plastique et grâce à la souplesse naturelle du ruban, le ruban s'enroule en demi-lune autour du doigt permettant d'obtenir une empreinte roulée de bonne qualité difficilement réalisable avec les systèmes déjà existants. Ces mêmes alvéoles, ainsi que la butée, permettent par ailleurs de contrôler la direction et la position du doigt au moment de la saisie, ce qui garantit la position et l'orientation de l'empreinte et par conséquent sa reproductibilité.

Un autre des avantages de ce système est la protection physique des empreintes apportée par l'adhésif qui, une fois retourné et collé sur le formulaire protège l'information acquise des agressions extérieures éventuelles (humidité, jaunissement ...).

La finesse du pigment noir (composé de mine de crayon plus ou moins grasse) donne, quant à elle, au dessin de l'empreinte une clarté et une qualité très recherchées ; cette dernière est telle que les pores de la peau se distinguent fréquemment à l'oeil nu.

Ce système a de plus l'avantage de ne pas engager un coût de fabrication important, ses composants eux-mêmes étant peu coûteux, faciles à se procurer et à maintenir.

En se reportant à la figure 6a, on va décrire une variante de réalisation dans laquelle le bloc support 1 est cylindrique. Le bloc support est monté sur un axe 20 et peut tourner autour de cet axe. Selon l'exemple de la figure 6, la périphérie du bloc support 1 est munie d'alvéole 3 à 3"". A la périphérie du bloc est également prévu un système de fixation 17 de l'extrémité du support. Ce système peut être tel que représenté en figure 6b réalisé à l'aide d'un clapet ou pince qui plaque le ruban contre le support. Pour permettre une rotation du support, celui est muni d'un évidement dans laquelle on place l'extrémité du support et dans laquelle le clapet vient se rabattre.

A la partie inférieure du support 1 est prévu un plateau support 16. Le support 1 est situé légèrement au-dessus du plateau 16, à une faible distance de telle façon qu'une feuille de papier de carton ou de plastique (par exemple) placée sur le plateau 16 soit entraînée par la rotation du support 1 et que le ruban autocollant se colle sur la feuille au fur et à mesure de la rotation du support et du déplacement de la feuille.

Un système à clapet est associé au bloc support 1 de façon à permettre la rotation pas à pas du support.

En position initiale l'alvéole 3 est à la partie support. Après la prise d'empreinte d'un premier doigt (l'index par exemple), le support est tourné d'un pas pour que l'alvéole 3' se trouve à la partie supérieure et ainsi de suite.

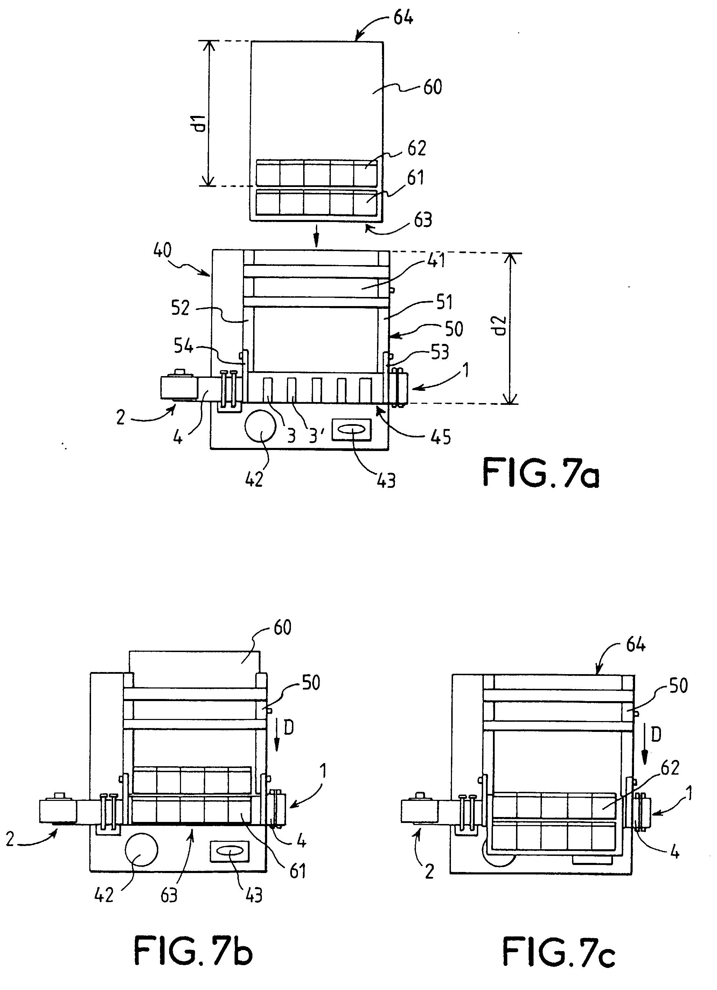

En se reportant aux figures 7a à 7c, on va maintenant décrire un système de mise en oeuvre de l'invention pour l'application d'empreintes digitales sur un document.

Dans ce système, on retrouve le dispositif de saisie d'empreintes digitales décrit précédemment et comprenant notamment le bloc support 1, le dévideur 2 et le ruban adhésif 4. Ce dispositif de saisie d'empreintes est solidaire d'une plate-forme 40 permettant la mise en place de document 60.

Un document 60 comporte un emplacement 61 pour recevoir les empreintes digitales d'une main (la main droite par exemple) et un emplacement 62 pour recevoir les empreintes de l'autre main. Le document comporte un bord avant 63 et un bord arrière 64.

La plate-forme 40 porte un chariot 50 comportant deux guides latéraux 51 et 52. Un document 60 peut être placé entre ces guides après que des empreintes digitales d'une main (la main droite par exemple) ont été prises sur le ruban adhésif 4. Un document 60 est placé entre les guides 51 et 52 avec son bord avant 63 affleurant le bord latéral 45 du ruban situé du côté opposé au chariot 50 par rapport au ruban 5. Le document est donc positionné convenablement que ce soit selon la direction latérale du document que selon sa direction longitudinale (voir figure 7b). Le document 60 est appliqué au ruban adhésif 4. Celui portant les empreintes se trouve donc à l'emplacement 61 du document. Ensuite, le chariot 50 est déplacé selon la flèche D. Les guides 51 et 52 du chariot comportent à leur extrémité située vers le ruban adhésif 4, des dispositifs de découpe (lames de rasoir par exemple) permettant lorsqu'on déplace le chariot, de découper le ruban adhésif 4 de part et d'autre du document 60. De préférence, ces dispositifs de découpe sont placés de façon à découper le ruban adhésif 4 au ras des bords latéraux du document.

Ensuite, le document 60 est retiré du système. Eventuellement, le collage du ruban adhésif sur le document est complété.

Une autre portion de ruban est placée sur le bloc support 1. On procède à la prise des empreintes digitales de l'autre main (main gauche) sur cette portion de ruban adhésif. Puis on replace le document 60 entre les guides 51 et 52. Le positionnement latéral du document est assuré par les guides comme précédemment. Par contre, le positionnement longitudinal est assuré en plaçant le bord arrière 64 du document au rebord de la partie de la plate-forme 44 situé du côté opposé au bord 45 du ruban par rapport au ruban lui-même. Au lieu du rebord 44, on peut prévoir le positionnement par rapport à des repères (non représentés) prévus sur la plate-forme. Quoi qu'il en soit, pour que la portion du ruban adhésif soit appliquée dans la zone 62 du document, le système est prévu pour que la distance d2 du rebord 44 de la plate-forme (ou des repères en tenant lieu) au bord 45 du ruban correspond à la distance d1 séparant le bord arrière 64 du document du bord de l'emplacement 62 le plus éloigné du bord 44 de la plate-forme.

Ensuite, lorsque le document a été appliqué au ruban adhésif (figure 7c), le chariot est déplacé selon la flèche D de façon à découper le ruban adhésif de part et d'autre du document. Enfin, le document est retiré du système et le collage du ruban adhésif est complété.

A titre complémentaire, la plate-forme comporte une zone dans laquelle est placée un récipient 42 contenant un pigment et permettant l'enduction des extrémités des doigts dont on doit prendre les empreintes, ainsi que des moyens 43 (tampon) pour enlever les pigments en excès et pour répartir ce pigment sur les empreintes.

## Revendications

1. Dispositif de saisie d'empreintes digitales comprenant :
- un bloc support (1) muni à sa face supérieure (12) d'au moins une alvéole (3) de forme allongée selon un axe ;
- un dévideur (2) de ruban autocollant situé à une première extrémité (10) du bloc et permettant le dévidage du ruban adhésif perpendiculairement à l'axe de l'alvéole, la face non collante du ruban reposant sur le bloc au-dessus de l'alvéole.

2. Dispositif selon la revendication 1, caractérisé en ce que le bloc support (1) comporte cinq alvéoles (3 à 3"") disposées parallèlement.

3. Dispositif selon l'une des revendications 1 ou 2, caractérisé en ce que les alvéoles sont de forme cylindrique.

4. Dispositif selon la revendication 1, caractérisé en ce qu'il comporte une butée (5) située à une extrémité de l'alvéole.

5. Dispositif selon la revendication 1, caractérisé en ce qu'il comporte un système d'immobilisation (9) du ruban, situé à une deuxième extrémité (11) du bloc support opposée à la première extrémité (10).

6. Dispositif selon la revendication 1, caractérisé en ce qu'il comporte un système de tension élastique (7) situé à la première extrémité (10) du bloc support entre le dévidoir et le bloc.

7. Dispositif selon la revendication 2, caractérisé en ce qu'il comporte des barres de séparation (6 à 6"") situées au-dessus du bloc support, entre les alvéoles et permettant le passage du ruban autocollant entre ces barres et la face supérieure (12) du bloc.

8. Dispositif selon l'une des revendications 4 ou 7, caractérisé en ce que les barres de séparation (6 à 6"") sont fixées sur la butée (5).

9. Dispositif selon la revendication 8, caractérisé en ce que la butée (5) et les barres de séparation (6 à 6"") sont amovibles.

10. Dispositif selon la revendication 9, caractérisé en ce que la butée (5) est articulée autour d'un axe de façon à pouvoir écarter la butée et les barres de séparation de la face supérieure du bloc support.

11. Dispositif selon la revendication 5, caractérisé en ce que le système d'immobilisation (9) comporte une barre d'immobilisation parallèle à l'axe des alvéoles et ayant deux positions :
- une position basse permettant d'appliquer le ruban contre la face supérieure (12) du support et de l'immobiliser ;
- une position haute permettant le déplacement du ruban.

12. Dispositif selon l'une des revendications 9 ou 11, caractérisé en ce que la barre d'immobilisation est solidaire de la butée (5).

13. Dispositif selon la revendication 1, caractérisé en ce qu'il comporte à la première extrémité (10) ou à la deuxième extrémité (11) du support, un système de découpe du ruban.

14. Dispositif selon l'une des revendications 11 ou 13, caractérisé en ce que la barre d'immobilisation est située entre l'alvéole la plus proche de la deuxième extrémité et le système de découpe.

15. Dispositif selon la revendication 13, caractérisé en ce que le système de découpe comporte une lame tranchante.

16. Dispositif selon la revendication 15, caractérisé en ce que le système de découpe est une lame en dents de scie.

17. Dispositif selon la revendication 14, caractérisé en ce qu'entre la barre d'immobilisation (9) et le système de découpe est prévu un espace (13) permettant de maintenir manuellement le ruban contre la face supérieure (12) du bloc support lorsqu'on écarte la barre d'immobilisation de la face supérieure.

18. Dispositif selon la revendication 13, caractérisé en ce qu'il comporte une lame amovible qui, en coopération avec l'extrémité (11) du bloc support, forme cisaille.

19. Dispositif selon l'une des revendications 9 ou 18, caractérisé en ce que la lame amovible est solidaire de la butée (5).

20. Dispositif selon la revendication 1, caractérisé en ce que le dévideur (2) de ruban autocollant comporte un frein de dévidage ou un dispositif de blocage du dévideur qui permet de freiner ou de bloquer la rotation du dévideur durant l'opération de prise d'empreintes.

21. Dispositif selon l'une des revendications 9 ou 15, caractérisé en ce que la lame tranchante est solidaire de la butée (5).

22. Dispositif selon l'une des revendication s1, 2 ou 13, caractérisé en ce que le bloc support (1) est de forme cylindrique, l'alvéole (3) est située à la périphérie de la forme cylindrique, et comporte des moyens d'entraînement (17) du ruban autocollant de façon que la rotation du support sur son axe entraîne le ruban.

23. Dispositif selon la revendication 22, caractérisé en ce que le bloc support (1) est situé à une distance telle d'un plateau support (16) qu'une feuille placée sur le plateau soit entraîné par le bloc support lors de sa rotation et que le ruban autocollant soit collé sur la feuille.

24. Procédé de prise d'empreintes digitales, caractérisé en ce qu'il prévoit d'enduire le doigt dont on veut prendre l'empreinte avec un pigment coloré, puis de l'appliquer sur la surface collante d'un ruban autocollant transparent et enfin de coller le ruban autocollant sur un support.

25. Procédé selon la revendication 24, caractérisé en ce que le ruban est placé sur une forme en creux ayant sensiblement la forme du doigt, la face non collante en contact avec la forme.

26. Dispositif selon la revendication 1, caractérisé en ce qu'il comporte une plate-forme (40) solidaire du bloc support (1), ladite plate-forme portant des guides (51, 57) de positionnement latéral de document (60) et permettant le placement d'un document au-dessus du bloc support en vue du collage du document à une portion de ruban adhésif porteur d'empreintes digitales.

27. Dispositif selon la revendication 26, caractérisé en ce qu'il comporte un chariot mobile (50) pouvant se déplacer le long des bords latéraux du document vers le ruban adhésif, ce chariot étant muni de moyens de découpe (53, 54) permettant de découper le ruban de part et d'autre du document lors d'un déplacement du chariot.

28. Dispositif selon la revendication 27, caractérisé en ce que le chariot mobile comporte lesdits guides (51, 52).

29. Dispositif selon la revendication 26, caractérisé en ce que la plate-forme comporte des repères situés à une distance (d2) par rapport au bord latéral (45) opposé du ruban qui est sensiblement égale à la distance (d1) d'un bord (64) du document (60) par rapport au bord d'un emplacement (62) du document destiné à recevoir une portion de ruban adhésif porteur d'empreintes.

30. Dispositif selon la revendication 26, caractérisé en ce que lesdits repères sont constitués par un bord (64) de la plate-forme.

31. Dispositif selon la revendication 26, caractérisé en ce que la plate-forme comporte un emplacement muni d'un récipient à pigments (42) et un tampon (43) d'essuyage des doigts et de répartition des pigments sur les doigts.
